# EUROPEAN PATENT APPLICATION

(11) **EP 3 470 083 A1**
(43) Date of publication of application: **17.04.2019**
(21) Application number: 17812423.6
(22) Date of filing: 27.03.2017
(51) Int. Cl.: A61K 47/04, A61K 49/00

(54) **COMPOSITION FOR MEDICAL APPLICATIONS FORMED THROUGH ION IMPLANTATION**

(30) Priority: 13.06.2016 US 201615180309
(71) Applicant: Niu, Huan, Taipei, Taiwan 10488 (TW); Chen, Chien-Hsu, Hsinchu, Taiwan 30064 (TW); Yi, Chih-Yang, Taoyuan 33376 (TW)
(72) Inventor: Niu, Huan, Taipei, Taiwan 10488 (TW); Chen, Chien-Hsu, Hsinchu, Taiwan 30064 (TW); Yi, Chih-Yang, Taoyuan 33376 (TW)
(74) Representative: Petraz, Gilberto Luigi
(86) International application number: PCT/CN2017/078338
(87) International publication number: WO 2017/215309

(57) **Abstract**

A composition (10) for medical applications and a preparation method therefor, the composition (10) including at least one magnetic nanoparticle (100) comprising a nanodiamond (110) and at least one magnetic element (120), said at least one magnetic element (120) being embedded in the at least one nanodiamond (110) by means of an ion implantation system (400). The nanodiamond (110) may be synthesized with different components so as to improve the medical efficacy of the described composition (10).

## Description

### FIELD OF THE INVENTION

The present invention relates to composition for medical usage and method of preparing the same, and more particularly to magnetic nanoparticles which are formed by ion implantation of magnetic elements into nanodiamond particles, wherein such magnetic nanoparticles can serve as a composition for further use of various medical purposes.

### BACKGROUND OF THE INVENTION

Recently, a field of research dedicated to the treatment of cancer patients based on the localized heating effects of magnetic nanoparticles in vivo has been developed. For many years, scientists have been doing research and reported the potential use of heat treatment which can cure or shrink tumor. Hyperthermia is being widely used in cancer treatment, which utilizes heat to destroy cancerous tumors. In hyperthermia therapy, i.e., hyperthermia in cancer treatment, the effects of heat on cancer cells are well-known, and electromagnetic fields have been used to localize and concentrate heat by directly heating the magnetic nanoparticles so as to cause a temperature increase within nearby cancer cells. Also, the outcome measured by patient survival and tumor regression is better when heat and radiation are combined. Normal cells typically recover faster than cancer cells when exposed to either heat or the combination of heat and radiation. Additionally, normal tissues have more blood flow than cancerous tissue so that they dissipate heat better, and this is thus fortunate for cancer treatment by heat or heat and radiation combination.

In the conventional hyperthermia therapy, magnetic iron oxide/iron nanoparticles (such as Fe₂O₃, Fe₃O₄, Fe, etc.) are employed to be synthesized with targeting drugs for hyperthermia therapy. The magnetic iron oxide/iron nanoparticles are guided to cancer cells, and then they are induced to generate heat by exposure to an alternating radiofrequency magnetic field such that the cancer cells heating came from hysteresis losses from the magnetic iron oxide/iron nanoparticles.

However, the above mentioned magnetic iron oxide/iron nanoparticles exhibit several disadvantages including cytotoxicity and poor biocompatible properties. Moreover, since the magnetic iron oxide/iron nanoparticle has few carbon-hydrogen bonds or ionic bonds on the surface thereof, it is difficult to synthesize a large amount of chemotherapy drugs or targeted agents with the magnetic iron oxide/iron nanoparticle. Accordingly, it is necessary to provide a new type of magnetic nanoparticles which have much less toxic side effects for medical usage and can easily synthesize with a large amount of chemotherapy drugs or target agents so as to solve the technical problems in the prior art.

### SUMMARY OF THE INVENTION

In order to solve the above-mentioned technical problems, an objective of the present invention is to provide a composition and a method of preparing the same. The composition comprises magnetic nanoparticles which are formed by embedding magnetic elements into nanodiamond particles by using ion implantation technique and such composition can be further synthesized with targeting agents and to deliver such the composition to tumor cells for hyperthermia therapy. Moreover, the magnetic nanoparticles in the composition can provide the use of magnetic resonance imaging to precisely diagnose tumor location and distribution. Thus, the delivery ability of compositions through the synthesized targeting agents can be monitored by using magnetic resonance imaging so as to determine whether enough concentration of compositions is reached at a target site in a patient for proper treatment. In addition, the composition can be further synthesized with other medicine agents, for example antimicrobial agent, such that the composition can be guided to concentrate at a targeted site in the patient by applying an external magnetic field so as to treat a bacterial infection or other diseases.

In order to achieve the above objective, the present invention provides a composition for medical usage, comprising: at least one magnetic nanoparticle including a nanodiamond particle and at least one magnetic element, wherein the at least one magnetic element is embedded into the at least one nanodiamond particle through ion implantation technique by using an ion implantation system.

In one preferred embodiment of the present invention, the at least one magnetic element is selected from the group consisting of at least one Iron (Fe), Manganese (Mn), Cobalt (Co), and Nickel (Ni) element.

In one preferred embodiment of the present invention, the composition further comprises at least one targeted agent synthesized with the nanodiamond particle of the at least one magnetic nanoparticle for targeted delivery of the composition to a target site in a subject to perform a hyperthermia therapy.

In one preferred embodiment of the present invention, the composition contains magnetic nanoparticle can provide the use of magnetic resonance imaging technique to precisely diagnose tumor location and distribution in an environment of the target site, such that a delivery ability of the composition through the targeting agent is monitored by using MRI so as to determine whether the composition and its required concentration has been delivered effectively to the target site in the subject for proper treatment.

In one preferred embodiment of the present invention, the composition further comprises at least one other medicine agent, for example antimicrobial agent, synthesized with the nanodiamond particle of the at least one magnetic nanoparticle, whereby the composition is suitable to be guided to concentrate at a target site of a patient by applying an external magnetic field.

In one preferred embodiment of the present invention, the ion implantation system comprising: an ion source assembly, for generating ions; an extraction assembly, for extracting the ions from the ion source assembly to form an ion beam; an analyzing magnet, for selecting the at least one magnetic element from the ion beam; and an end station, for supporting a workpiece which holds the nanodiamond particle and located in a path of the ion beam such that the at least one magnetic element is implanted into the workpiece thereby to embed the at least one magnetic element into the nanodiamond particle.

In one preferred embodiment of the present invention, the ion implantation system comprises a plasma immersion ion implantation.

The present invention further provides a method of preparing a composition for medical usage, comprising: providing a workpiece which holds at least one nanodiamond particle; disposing the workpiece on an ion implantation system; and embedding at least one magnetic element into the at least one nanodiamond particle to form a magnetic nanoparticle to obtain the composition by using the ion implantation system.

In one preferred embodiment of the present invention, the method further comprises: synthesizing at least one targeted agent with the at least one nanodiamond particle of the at least one magnetic nanoparticle to obtain the composition whereby the composition is suitable for targeted delivery to a target site in a subject to perform a hyperthermia therapy.

In one preferred embodiment of the present invention, the method further comprises: synthesizing at least one medicine agent with the at least one nanodiamond particle of the at least one magnetic nanoparticle to obtain the composition, whereby the composition is suitable to be guided to concentrate at a target site of a patient by applying an external magnetic field.

In one preferred embodiment of the present invention, the at least one medicine agent comprises at least one antimicrobial agent.

In one preferred embodiment of the present invention, the step of embedding at least one magnetic element into the at least one nanodiamond particle to form a magnetic nanoparticle by using the ion implantation system comprises: generating ions by an ion source assembly of the ion implantation system; extracting the ions from the ion source assembly to form an ion beam by an extraction assembly of the ion implantation system; selecting the at least one magnetic element from the ion beam by an analyzing magnet of the ion implantation system; and implanting the at least one magnetic element into the workpiece so as to embed the at least one magnetic element into the at least one nanodiamond particle.

In one preferred embodiment of the present invention, after the step of implanting the at least one magnetic element into the workpiece, the method further comprises: annealing the workpiece.

The present invention further provides a usage of the aforementioned composition for the preparation of a medicament having at least one of following effects: treating cancer, diagnosing cancer, and treating bacterial infection.

### BRIEF DESCRIPTION OF THE DRAWINGS

In order to illustrate the technical solutions in the embodiments of the present invention or in the prior art more clearly, the following briefly introduces the accompanying drawings required for describing the embodiments or the prior art. The accompanying drawings in the following description are merely some embodiments of the present invention, and persons of ordinary skill in the art may still derive other drawings from these accompanying drawings without creative effort.
Fig. 1A shows a schematic diagram of a composition according to a first preferred embodiment of the present invention.
Fig. 1B shows an enlarged view of a portion of Fig. 1A.
Fig. 1C is a schematic diagram showing magnetic elements implanted into a nanodiamond particle for obtaining the composition of Fig. 1A.
Fig. 2 shows a schematic diagram of a composition according to a second preferred embodiment of the present invention.
Fig. 3 shows a schematic diagram of the magnetic nanoparticle of the composition further synthesized with medicine agents.
Fig. 4 depicts a schematic diagram showing an ion implantation system for performing a method of preparing magnetic nanoparticles according to a preferable embodiment of the present invention.
Figs. 5A and 5B illustrate schematic diagrams respectively showing nanoparticles obtained from a workpiece of the ion implantation system of Fig. 4, and how to separate magnetic nanoparticles and non-magnetic nanoparticles of the nanoparticles of FIG. 5A.
Fig. 6 depicts a schematic diagram showing a method of preparing a composition by using a plasma immersion ion implantation.

### DETAILED DESCRIPTION OF THE INVENTION

Please refer to the accompanying drawings, similar parts are denoted with the same reference numerals. The following description is based on the particular embodiments of the present invention, and they should not be construed as limiting the invention to the other embodiments which are not discussed in detail herein.

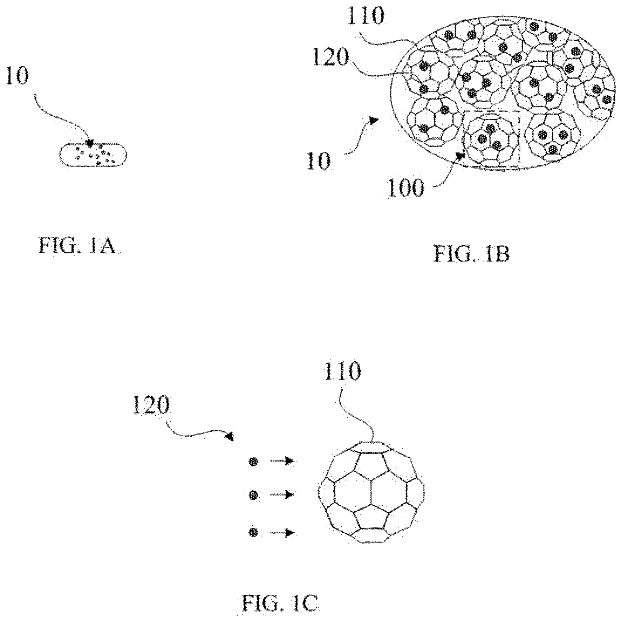

Please refer to Fig. 1A, which shows a schematic diagram of a composition 10 according to a first preferred embodiment of the present invention. Fig. 1B shows an enlarged view of a portion of the composition 10 and Fig. 1C is a schematic diagram showing magnetic elements 120 implanted into a nanodiamond particle 110 for obtaining the composition 10 of Fig. 1A. As shown on Fig. 1B, the composition 10 comprises a plurality of nanodiamond particles 110 and a plurality of magnetic elements 120, where the magnetic elements 120 are selected from atoms having ferromagnetism, such as Iron (Fe), Manganese (Mn), Cobalt (Co), or Nickel (Ni) elements. Moreover, as shown on Fig. 1B and Fig. 1C, the magnetic elements 120 are directly implanted into each nanodiamond particle 110 by using an ion implantation system, so that the plurality of magnetic elements 120 are embedded into the nanodiamond particle 110 so as to form a magnetic nanoparticle 100.

It should be noted that, in the present invention, since the nanodiamond particles 110 employed exhibit no obvious cytotoxicity to human cells, and do not induce significant abnormality in cellular functions, the composition 10 is of particular benefit in biomedical and medical applications, e.g. drug delivery, therapy, and diagnostic techniques. However, the scope of the present invention is not limited to the nanodiamond particle only; any other nanoparticles which exhibit no obvious toxicity and have excellent biocompatibility for cancer diagnosis and therapies as well can be selected and within the scope of the present invention. Furthermore, since the nanodiamond particle 110 has a large loading capacity for synthetizing with various types of agents/drugs due to its large surface area to volume ratio, it is advantageous to modify and utilize surface functional properties of the nanodiamond particle 110 to conjugate with therapeutic agents for a specific purpose or multiple purposes usage, such as to deliver a drug with localized distribution to cancer cells, limited diffusion to increase uptakes in cancer cells, image capability to locate cancer cells precisely, and increased drug retention period for cancer treatment. In addition, the surface of the magnetic nanoparticles 100 can be modified to form bonds with folate moieties to effectively target cancer cell which usually is over-expressed with folate receptors than normal cell. So that the cancer cells targeted by the magnetic nanoparticles 100 bonded with folate moieties can have high death rate after hyperthermia therapy.

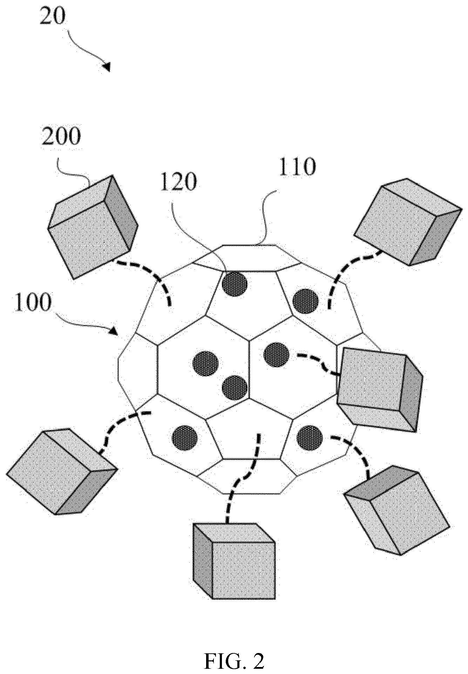

Please refer to Fig. 2, which shows a schematic diagram of a composition 20 according to a second preferred embodiment of the present invention. The composition 20 comprises at least one magnetic nanoparticle 100, in which the nanodiamond particle 110 of the magnetic nanoparticle 100 is further synthesized with targeted agents 200. Due to the magnetic elements 120 have ferromagnetism, the composition 20 is suitable for use in hyperthermia therapy, where the nanodiamond particle 110 serves as a carrier for in vivo delivery of the magnetic elements 120 to a target site in a subject including a human being or an animal. To be more specific, to perform the hyperthermia therapy in the subject includes the following steps. Firstly, the composition 20 is administered to the subject, and the magnetic nanoparticles 100 will be introduced close to or within the target site by the targeted agents 200. Next, after the magnetic nanoparticles 100 are located at the target site, the target site is exposed to an electromagnetic field effective to generate heat from the magnetic nanoparticles 100 so as to cause a temperature increase within the target site to destroy cancer cells thereof. In addition, in the present invention, since the magnetic nanoparticles 100 of the composition 20 include the magnetic elements 120, the magnetic nanoparticles 100 can provide the use of magnetic resonance imaging technique to precisely diagnose tumor location and distribution in a patient. Thus, an environment of the target site and the delivery ability of composition 20 through the synthesized targeted agents 200 can be monitored by using magnetic resonance imaging (MRI) so as to determine whether the composition 20 and its desired concentration has been delivered effectively and reached the target site in the subject for proper treatment. Methods of magnetic resonance imaging in the subject are well-known in the art. It should be noted that since the composition 20 includes the magnetic elements 120 which have superior MRI signal enhancement capabilities in vivo, the composition 20 also can be used to diagnose other diseases as well.

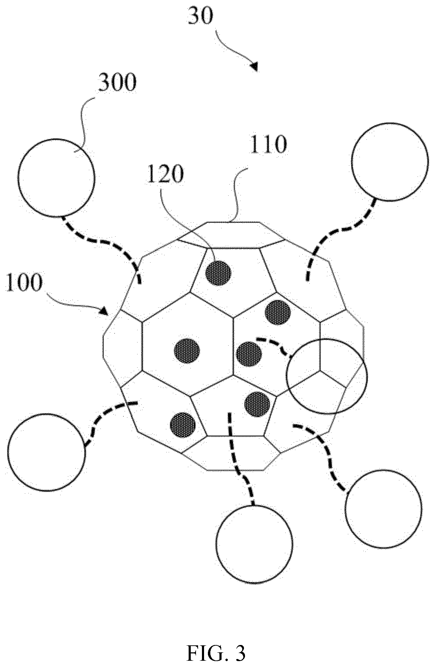

On the other hand, in order to ensure that the administered drugs including medicine agents (such as antimicrobial agents) can be successfully concentrated at a correct location in a subject (i.e., human being), the drugs can be synthesized with the magnetic nanoparticle 100 of the present invention. Please refer to Fig. 3, which shows a schematic diagram of a composition 30 according to a third preferred embodiment of the present invention. The composition 30 comprises at least one magnetic nanoparticle 100, in which the nanodiamond particle 110 of the magnetic nanoparticle 100 is further synthesized with medicine agents 300. Since the magnetic elements 120 have ferromagnetism, the composition 30 can be easily guided into a desired location through applying an external magnetic field to the desired location. Therefore, the delivery efficiency of the medicine agents 300 can be increased, and a total intake amount of drug (i.e., the composition 30) can be reduced so as to prevent side effects associated with administration of the medicine agents 300. In addition, if the magnetic nanoparticle 100 is further synthesized with antimicrobial agents, the composition 30 is suitable for treating a bacterial infection.

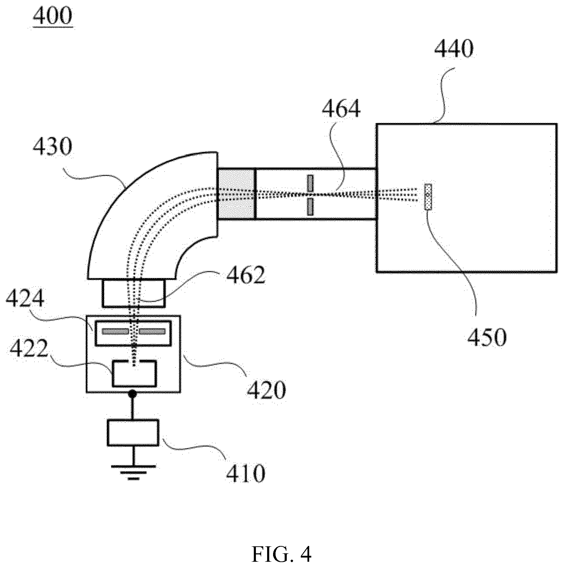

Please refer to Fig. 4, which depicts a schematic diagram showing an ion implantation system 400 for performing a method of preparing magnetic nanoparticles according to a preferable embodiment of the present invention. The ion implantation system 400 includes a high voltage power supply 410, an ion source assembly 420, an arc chamber 422, an extraction assembly 424, an analyzing magnet 430, and an end station 440. The method of preparing the magnetic nanoparticles according to the present invention includes the following steps. Firstly, a workpiece 450 which holds nanodiamond particles is provided and disposed at the end station 440 of the ion implantation system 400. Specifically, the workpiece 450 holding nanodiamond particles is manufactured by performing the steps of: dissolving nanodiamond powder in deionized water to form a solution; dropping the solution on a substrate (e.g. an oxidized silicon wafer); and removing the deionized water from the substrate by heating the substrate under a lamp, so as to form the workpiece 450 holding the nanodiamond particles.

After the workpiece 450 holding the nanodiamond particles is disposed at the end station 440, the ion implantation system 400 is switched on, and ions are generated by the ion source assembly 420 from feeding materials into the arc chamber 422. Next, the ions are extracted from the ion source assembly 420 to form a first ion beam 462 by the extraction assembly 424 which provides electrical potential difference between the ion source assembly 420 and the extraction assembly 424, so that the first ion beam 462 gains energy to leave the ion source assembly 420 toward downstream.

When the first ion beam 462 is directed into the analyzing magnet 430, the desired magnetic elements are selected from the first ion beam 462 by the analyzing magnet 430, and a second ion beam 464 including the magnetic elements is thus formed and directed from the analyzing magnet 430 into the end station 440. In the end station 440, the workpiece 450 holding the nanodiamond particle is located in a path of the second ion beam 464. Therefore, after the second ion beam 464 is guided into the end station 440, the workpiece 450 is implanted by the second ion beam 464 with a controlled final energy to embed the magnetic elements into the nanodiamond particles such that the magnetic nanoparticles are thus formed.

It should be noted that, the crystal lattice structure of the nanodiamond particle may be damaged after performing the ion implantation process, so an annealing process is employed to repair the damage of the nanodiamond particle. To be specific, after the magnetic elements are implanted into the nanodiamond particles, the workpiece 450 is annealed. Moreover, the crystal lattice structure of the nanodiamond particle being successfully repaired by performing the annealing process can be proven by employing Raman spectroscopy to examine the structural changes of the nanodiamond particle before performing the ion implantation process, after performing the ion implantation process, and after further performing the annealing process. For example, according to the experimental result, the original nanodiamond particles have a diamond peak at 1332.5 cm⁻¹ and a broad peak at 1579 cm⁻¹, which is probably due to surface graphitic structure. The full width at half maximum (FWHM) of the diamond peak is 10.1 cm⁻¹. After implanting Fe ions into the nanodiamond particles, the diamond peak is shifted to 1317 cm⁻¹ with a much wider FWHM of 98.5 cm⁻¹. The blue shift of the Raman peak and the widening of the FWHM are obviously indicative of that the crystal lattice structure of the nanodiamond particle is damaged. Furthermore, after annealing, the diamond peak become very strong and is restored to the original position at 1332.4 cm⁻¹ and the FWHM is reduced to 11.1 cm⁻¹. Also, the other peak related to the surface sp² bonds is disappeared. The result clearly indicates that the diamond structure is restored and the unwanted surface graphitic structure or its compounds are eliminated concurrently.

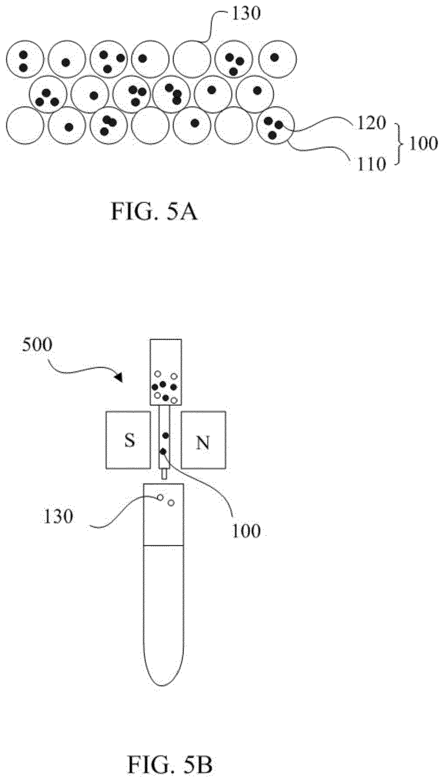

Please refer to Figs. 5A and 5B, which illustrate schematic diagrams respectively showing nanoparticles obtained from the workpiece 450 of the ion implantation system 400 of Fig. 4, and how to separate magnetic nanoparticles 100 and non-magnetic nanoparticles, i.e., non-doped nanodiamond particles 130 of the nanoparticles of FIG. 5A. As shown on Fig. 5A, after the magnetic elements 120 are embedded into the nanodiamond particles 110 to form the magnetic nanoparticles 100, the magnetic nanoparticles 100 and some non-doped nanodiamond particles 130 are removed from the substrate of the workpiece 450 by using an ultrasonic bath of deionized water. In this embodiment, since the nanodiamond particles 110 are stacked on the substrate (e.g. Si wafer) with multi-layers, only a part of the nanodiamond particles 110 will be implanted by the magnetic elements 120. As shown on Fig. 5B, in order to sort out the magnetic nanoparticles 100, a magnetic filter 500 can be employed. The magnetic filter 500 may be a magnetic-activated cell sorting tool (MACS® Separator, Miltenyi Biotec Co.). The magnetic nanoparticles 100 embedded with the magnetic elements 120 are attracted by the magnet filter 500 and gathered to the adjacent walls when the nanodiamond solution, which is prepared by dissolving the removed nanoparticles from the workpiece 250 in deionized water, passes through the magnetic filter 500. Also, the non-doped nanodiamond particles 130 will directly pass freely through the magnetic filter 500 in downward direction. Then the attached magnetic nanoparticles 100 can be flushed out by injected deionized water. Finally, the magnetic nanoparticles 100 are obtained and they can be collected in a beaker with deionized water.

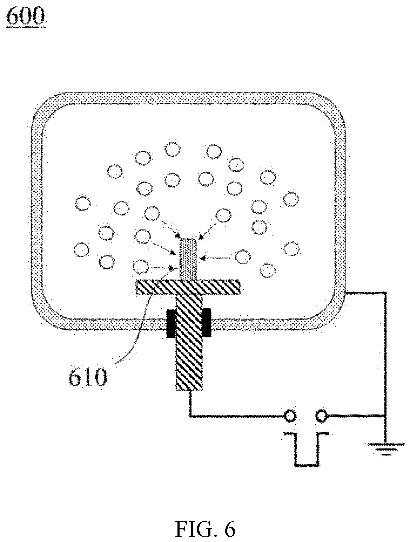

Please refer to Fig. 6, which depicts a schematic diagram showing a method of preparing a composition by using a plasma immersion ion implantation 600. In this embodiment, the plasma immersion ion implantation 600 is used for embedding magnetic elements into nanodiamond particles which are located on a bombardment target 610 of the plasma immersion ion implantation 600.

In summary, in the present invention, the magnetic nanoparticles of the composition are prepared by implanting magnetic elements into nanodiamond particles using ion implantation technique for medical usage such that the disease can be effectively treated without exhibiting cytotoxicity to normal cells due to the magnetic elements are embedded with the non-toxic nanodiamond particles. For example, the magnetic nanoparticles of the composition can be further synthesized with targeted agents, whereby the composition can be precisely delivered to tumor cells for hyperthermia therapy. Moreover, the magnetic nanoparticles of the composition can increase provide the use of magnetic resonance imaging to precisely diagnose tumor location and distribution in a patient. Thus, the delivery ability of the composition through the synthesized targeted agents for tumor treatment can be monitored by using magnetic resonance imaging directly so as to determine whether enough magnetic nanoparticles have reached a target site in a patient for proper treatment. In addition, the composition can be further synthesized with medicine agents, e.g. antimicrobial agents, such that the composition can be guided to concentrate at a target site in the patient by applying an external magnetic field so as to treat a bacterial infection or other diseases.

The above descriptions are merely preferable embodiments of the present invention, but are not intended to limit the scope of the present invention. Any modification or replacement made by those skilled in the art without departing from the spirit and principle of the present invention should fall within the protection scope of the present invention. Therefore, the protection scope of the present invention is subject to the appended claims.

## Claims

1. A composition for medical usage, **characterized in that** the composition comprises: at least one magnetic nanoparticle including a nanodiamond particle and at least one magnetic element, wherein the at least one magnetic element is embedded into the at least one nanodiamond particle by using an ion implantation system.

2. The composition for medical usage as claimed in claim 1, **characterized in that** the at least one magnetic element is selected from the group consisting of at least one Iron (Fe), Manganese (Mn), Cobalt (Co), and Nickel (Ni) element.

3. The composition for medical usage as claimed in claim 1, **characterized in that** the composition further comprises at least one targeted agent synthesized with the nanodiamond particle of the at least one magnetic nanoparticle for targeted delivery of the composition to a target site in a subject to perform a hyperthermia therapy.

4. The composition for medical usage as claimed in claim 3, **characterized in that** a delivery ability of the composition through the targeting agent is monitored by using magnetic resonance imaging (MRI) so as to determine whether the composition has reached the target site with a desired concentration.

5. The composition for medical usage as claimed in claim 1, **characterized in that** the composition further comprises at least one medicine agent synthesized with the nanodiamond particle of the at least one magnetic nanoparticle, whereby the composition is suitable to be guided to concentrate at a target site of a patient by applying an external magnetic field.

6. The composition for medical usage as claimed in claim 1, **characterized in that** the at least one medicine agent comprises at least one antimicrobial agent.

7. The composition for medical usage as claimed in claim 1, **characterized in that** the ion implantation system comprising:
an ion source assembly, for generating ions;
an extraction assembly, for extracting the ions from the ion source assembly to form an ion beam;
an analyzing magnet, for selecting the at least one magnetic element from the ion beam; and
an end station, for supporting a workpiece which holds the nanodiamond particle and located in a path of the ion beam such that the at least one magnetic element is implanted into the workpiece thereby to embed the at least one magnetic element into the nanodiamond particle.

8. The composition for medical usage as claimed in claim 1, **characterized in that** the ion implantation system comprises a plasma immersion ion implantation.

9. A method of preparing a composition for medical usage, **characterized in that** the method comprises:
providing a workpiece which holds at least one nanodiamond particle;
disposing the workpiece on an ion implantation system; and
embedding at least one magnetic element into the at least one nanodiamond particle to form a magnetic nanoparticle to obtain the composition by using the ion implantation system.

10. The method of preparing a composition for medical usage as claimed in claim 9, **characterized in that** the at least one magnetic element is selected from the group consisting of at least one Iron (Fe), Manganese (Mn), Cobalt (Co), and Nickel (Ni) element.

11. The method of preparing a composition for medical usage as claimed in claim 9, **characterized in that** the method further comprises: synthesizing at least one targeted agent with the at least one nanodiamond particle of the at least one magnetic nanoparticle to obtain the composition whereby the composition is suitable for targeted delivery to a target site in a subject to perform a hyperthermia therapy.

12. The method of preparing a composition for medical usage as claimed in claim 9, **characterized in that** the method further comprises: synthesizing at least one medicine agent with the at least one nanodiamond particle of the at least one magnetic nanoparticle to obtain the composition, whereby the composition is suitable to be guided to concentrate at a target site of a patient by applying an external magnetic field.

13. The method of preparing a composition for medical usage as claimed in claim 12, **characterized in that** the at least one medicine agent comprises at least one antimicrobial agent.

14. The method of preparing a composition for medical usage as claimed in claim 9, **characterized in that** the step of embedding at least one magnetic element into the at least one nanodiamond particle to form a magnetic nanoparticle by using the ion implantation system comprises:
generating ions by an ion source assembly of the ion implantation system;
extracting the ions from the ion source assembly to form an ion beam by an extraction assembly of the ion implantation system;
selecting the at least one magnetic element from the ion beam by an analyzing magnet of the ion implantation system; and
implanting the at least one magnetic element into the workpiece so as to embed the at least one magnetic element into the at least one nanodiamond particle.

15. The method of preparing a composition for medical usage as claimed in claim 14, **characterized in that** after the step of implanting the at least one magnetic element into the workpiece, the method further comprises: annealing the workpiece.

16. A usage of a composition according to any one of claims 1 to 8 for a preparation of a medicament having at least one of following effects: treating cancer, diagnosing cancer and treating bacterial infection.
